(19) Europäisches Patentamt
European Patent Office
Office européen des brevets

(11) EP 0 973 785 B1

(12) **EUROPEAN PATENT SPECIFICATION**

(45) Date of publication and mention of the grant of the patent:
**03.12.2003 Bulletin 2003/49**

(21) Application number: **98912826.9**

(22) Date of filing: **09.04.1998**

(51) Int Cl.[7]: **C07F 15/00**, C07H 23/00, G01N 33/543

(86) International application number:
**PCT/NL98/00206**

(87) International publication number:
**WO 98/045304 (15.10.1998 Gazette 1998/41)**

# (54) TRANS-PLATINUM COMPOUND, AND DIAGNOSTIC KIT

TRANS-PLATINVERBINDUNG UND DIAGNOSTISCHER TESTSATZ

COMPOSE A BASE DE TRANS-PLATINE ET NECESSAIRE DE DIAGNOSTIC

(84) Designated Contracting States:
**AT BE CH DE DK ES FI FR GB GR IE IT LI NL PT SE**

(30) Priority: **10.04.1997 EP 97201066**

(43) Date of publication of application:
**26.01.2000 Bulletin 2000/04**

(73) Proprietor: **Kreatech Biotechnology B.V.**
**1032 LG Amsterdam (NL)**

(72) Inventors:
- **HOUTHOFF, Hendrik, Jan**
  **NL-1023 NB Amsterdam (NL)**
- **REEDIJK, Jan**
  **NL-2334 CD Leiden (NL)**
- **VOLKERS, Herman, H.**
  **NL-1141 SL Monnickendam (NL)**
- **HEETEBRIJ, Robert, Jochem**
  **NL-3511 LT Utrecht (NL)**

(74) Representative: **Prins, Adrianus Willem et al**
**Vereenigde,**
**Nieuwe Parklaan 97**
**2587 BN Den Haag (NL)**

(56) References cited:
**WO-A-91/03482** **WO-A-95/07698**
**WO-A-96/35696** **US-A- 5 521 289**

- **CHEMICAL ABSTRACTS, vol. 66, no. 22, 29 May 1967 Columbus, Ohio, US; abstract no. 99016, GRINBERG, A. A. ET AL: "Water solubility of isomeric diammineplatinum compounds" XP002037092 & ZH. NEORG. KHIM. (1967), 12(1), 276-7 CODEN: ZNOKAQ, 1967,**
- **GOGGIN, P.L. ET AL.: "trimethylamine complexes of platinum(II) and palladium(II) and their vibrational and proton nuclear magnetic resonance spectra" JOURNAL OF THE CHEMICAL SOCIETY, DALTON TRANSACTIONS, 1972, pages 1298-1303, XP002037091**
- **CHEMICAL ABSTRACTS, vol. 120, no. 21, 23 May 1994 Columbus, Ohio, US; abstract no. 264838, KOSTIC, NENAD M.: "Transition-metal complexes as spectroscopic probes for selective covalent labeling and crosslinking of proteins" XP002037093 & METHODS ENZYMOL. (1993), 226(METALLOBIOCHEMISTRY, PT. C), 565-76 CODEN: MENZAU;ISSN: 0076-6879, 1993,**
- **CHEMICAL ABSTRACTS, vol. 115, no. 25, 23 December 1991 Columbus, Ohio, US; abstract no. 280550, ZHOU, XIA YING ET AL: "Steric effects on the selectivity of protein labeling with chelate complexes of platinum(II)" XP002037094 & POLYHEDRON (1990), 9(15-16), 1975-83 CODEN: PLYHDE;ISSN: 0277-5387, 1990,**
- **CHEMICAL ABSTRACTS, vol. 117, no. 23, 7 December 1992 Columbus, Ohio, US; abstract no. 227244, KISELEVA, V. I. ET AL: "Trans-diamminedichlorplatinum(II)-modifie d probes for detection of picogram quantities of DNA" XP002037095 & ANAL. BIOCHEM. (1992), 206(1), 43-9 CODEN: ANBCA2;ISSN: 0003-2697, 1992,**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

(Cont. next page)

- **CHEMICAL ABSTRACTS, vol. 108, no. 9, 29 February 1988 Columbus, Ohio, US; abstract no. 68422, SUNDQUIST, W.I. ET AL.: "monoclonal antibodies to dna modified with cis- or trans-diamminedichloroplatinum(II)" XP002037096 & PROC. NATL. ACAD. SCI. U.S.A., 1987, pages 8225-8229,**
- **BIOSIS AN: PREV199497548090 & Radiation Research; 1994, pages 55-62 Howell et al. Radiotoxicity of platinum-195m-labeled trans-platinum (II) in mammalian cells.**

## Description

The invention relates to a trans-platinum based compound, to a diagnostic kit comprising said compound, and to a method for labeling a bio-organic molecule wherein use is made of said compound.

Platinum (coordination) compounds have been considered interesting molecules for a very long time. For a review of these compounds and their uses we refer to Reedijk et al. (Structure and Bonding, 67, pp. 53-89, 1987). Especially cis-platinum has received a lot of attention as an anti-tumor drug. This anti-tumor reactivity of platinum compounds originates from their having at least two reactive groups (preferable cis-oriented towards each other), which make it possible to cross-link DNA molecules, thereby inhibiting the replication of these DNA molecules.

A different use of cis-platinum compounds has been described in international patent application WO-A-96/35696. Herein a method is disclosed for linking bio-organic molecules and labels through cis-platinum compounds, of which two coordination sites are occupied by two ends of a stabilizing bridge, such as an ethylene diamine group.

These known cis-platinum compounds are suitable for linking labels to several kinds of bio-organic molecules. Examples are (poly)-peptides, polynucleic acids and nucleotides.

In Anal. Biochem. 20b. 43-49 (1992), Kiseleva et al. have disclosed a method for detection of specific nucleotide sequences using a DNA-probe modified with trans-diamminedichlorplatinum (II).

The labelin g of polynucleic acids, such as DNA molecules, is desirable for applications in fields such as recombinant DNA technology. In many cases, however, one wishes not to label the nucleic acid macromolecule, but to label a certain nucleotide and enzymatically build this into a polynucleic acid. This way, the location of the label on the resulting polynucleic acid can be influenced, which is not possible when a label is attached to the macromolecule.

It has been found, however, that nucleotides linked to a label by cis-platinum linkers are not satisfactorily built in into DNA molecules by DNA polymerase, if at all. Therefore, in order to use a cis-platinum linker for obtaining a labeled DNA molecule one has to label the macromolecule.

The available alternatives for labeling a nucleotide, which can be incorporated into a polynucleic acid are the more conventional methods of labeling. However, these conventional methods also have a major disadvantage as they are not suitable for labeling any nucleotide. In some cases, for instance when only a few residues of a certain nucleotide are present in a certain polynucleic acid, or when the terminating nucleotide residue of a polynucleic acid is to be labeled, it is desired to be able to label any nucleotide.

An example of such a conventional method has been described by Dale et al., Biochemistry, **14**, (1975), 2447-2457, which method involves direct covalent mercuration as a labeling technique. Dale et el. report that cytosine and uracil may be mercurated at their C5-position under mild conditions. However, they also report that for adenine, thymine and guanine bases negative results were obtained.

Thus, there is a need for a universal linking system, which is excellent for linking labels and bio-organic molecules, including all different nucleotides, and which also makes it possible to enzymatically build in any nucleotide labeled through said linking system in a polynucleic acid in an efficient manner.

The present invention provides such a universal linking system. It has been found that a trans-platinum based compound, having the formula:

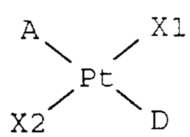

(I)

wherein A represents a reactive moiety for attachment to a label, D represents a reactive moiety for attachment to a bio-organic molecule, and X1 and X2 represent the same or different inert moieties, meets the above requirements for use in a method for labeling a bio-organic molecule.

Surprisingly, linking labels to several kinds of bio-organic molecules by using the compound of the invention is at least as efficient as the known methods. Further, it has been found that nucleotides linked to a label by the compound of the invention may be built in into polynucleotides very efficiently.

Nucleotides can assume two conformations: the syn-conformation and the anti-conformation. Definitions of these conformations can be found in Saenger, "Principles of Nucleic Acid Structure", Springer-Verlag Inc., 1984. It is believed that for a nucleotide to be satisfactorily built in into e.g. a DNA molecule it has to be in an anti-configuration. In US-A-4,711,955 it has been reported that altering of the normal anti-conformation of a nucleoside must be avoided, because otherwise the nucleotide derivatives are unacceptable as polymerase substrates. Thus nucleotides having a syn-conformation will not be enzymatically incorporated into a polynucleotide in an efficient manner, if at all.

Besides this, it is known from the above-mentioned publication by Saenger that the presence of a bulky group or substituent at the N7 position of a nucleotide, i.e. the position to which a metal coordinates most easily, induces a

nucleotide to have a syn-conformation. Thus, it would be expected that a nucleotide having a platinum compound coordinated to its N7 position would be in a syn-orientation. Accordingly, it would be expected to be not possible to incorporate said nucleotide into a polynucleotide with good results.

It is therefore very surprising that nucleotides linked to a label by a trans-platinum based compound in accordance with the invention may be built in into polynucleotides in a highly efficient manner.

The moieties X1 and X2 in formula (I) are inert. This means, that these moieties are ligands that remain bonded to the platinum core during the labeling reactions, i.e. they are stable under physiological conditions. They may be different or the same and may be any group that shows no interfering interactions between them and the label, bio-organic molecule or any other compound to be used in the application of the labeling techniques.

In these moieties X1 and X2 resides another advantage of the invention compared to the cis-platinum linkers. In order to obtain a stable cis-platinum compound it is imperative that said compound comprises a stabilizing bridge. For the trans-platinum compounds such a stabilizing bridge is not necessary. Accordingly, the possible variations in structure for the trans-platinum compounds of the invention are more numerous. Thus, according to the invention the skilled person has many instruments at his disposal for obtaining the desired properties of the compound.

In a preferred embodiment, X1 and X2 are the same or different non-leaving ligands. This means, that they are groups which have a very small leaving group character. Most preferred examples thereof are $NH_3$, $NH_2R$, NHRR', and NRR'R" groups, wherein R, R' and R" either represent an alkyl group having from 1 to 6 carbon atoms, as these groups form very stable bonds to the platinum core.

Particularly preferred is the embodiment wherein X1 and X2 are both an $N(CH_3)_3$ group. The presence of the methyl groups has a positive effect in that they prevent conformational modifications of the substrate due to for instance hydrogen bonding of a label or bio-organic molecule to the inert moieties.

The reactive moieties A and D are chosen such that they are easily replaced by or substituted with a label, respectively a bio-organic molecule. It is preferred that A and D are chosen from the group of acetate, $NO_3^-$, $HCO_3^-$, $CO_3^{2-}$, $SO_3^-$, $Cl^-$, $I^-$, and other halogens. These groups are good leaving groups and are therefore easily replaced.

In a highly preferred embodiment, a spacer, such as an oligolysine or a polylysine, is used. In accordance with this embodiment, A and/or D in formula (I) comprises a spacer, which spacer comprises a chain having at least four atoms, which chain comprises an electron donating moiety on one end and a reactive moiety on the other end, wherein the chain is attached to the platinum through the electron donating moiety. To the spacer, a label or a bio-organic molecule may be attached by a reaction with the end of the chain which is the most distant end seen from the platinum.

The use of a spacer has the advantage that the distance between the label and the bio-organic molecule can be optimized, so that steric factors cannot be an obstacle to an efficient labeling reaction.

The electron donating moiety of the spacer is preferably an amine group or a thiolate anion, because it has been found that these groups are capable of forming very strong bonds to platinum.

In a further preferred embodiment, the chain of the spacer comprises at least one heteroatom, preferably an oxygen atom, as this positively effects the hydrophilicity of the chain, which is an important aspect in physiological systems.

It is also possible to use a spacer that comprises a platinum atom. In this embodiment, the invention provides a bis-platinum compound for linking labels and bio-organic molecules. The second platinum can either have a cis or a trans orientation.

The spacer comprises preferably no more than 20 carbon atoms in the chain, which is preferably a non-branched chain, thus causing no steric hindrance. The reasons for this will be clear.

A highly preferred spacer is 1,8-diamino-3,6-dioxaoctane, herein referred to as Dadoo. Dadoo is a very flexible spacer having two primary amine groups and a size that makes it very suitable for use as a spacer according to the invention.

The labels that may be linked to a bio-organic molecule by the compound of the invention are not critical. In principle all labels which can be attached to a bio-organic molecule and are employed to date can be used. These labels may be radioactive labels, enzymes (which need reaction with a substrate to be detected), specific binding pairs components such as avidin, streptavidin or biotin, biocytin, iminobiotin, colloidal dye substances, fluorochromes (rhodamin, etc.) including Cy®-colourants, reducing substances (eosin, erythrosin, etc.), (coloured) latex sols, digoxigenin, metals (ruthenium), metal sols or other particulate sols (selenium, carbon and the like), dansyl lysin, Infra Red Dyes, coumarines (amino methyl coumarine), antibodies, protein A, protein G, etc. The invention has most benefits with bulkier labels such as biotin, avidin, streptavidin, and digoxygenin.

Cy®-colourants are preferred labels to be linked to bio-organic molecules by the compound of the invention. These labels can very suitably be used for a technique referred to as multi-colour labeling. The reason for this is that different colourants of this kind, while having different colours, are very much alike in chemical structure.

Almost every bio-organic molecule which contains an accessible sulfur or nitrogen atom can be linked to a label by the present compounds. Very suitable compounds to be labeled are proteins, peptides, DNA molecules, RNA

molecules, and (oligo)-nucleotides.

The platinum binds very easily to the N7 position of nucleotides. This way DNA or RNA molecules, be it single stranded or otherwise can be easily detected. It also allows for the production of probes for hybridization techniques wherein unlabeled DNA/RNA molecules hybridize to the labeled probe. The platinum compounds do hardly interfere with the hybridization, if at all.

The platinum compounds described herein are also very suitable for attaching bio-organic molecules to solid surfaces such as nitrocellulose, nylon filters or microtiter plates.

Nucleotides modified by using a linker according to the invention and oligo- and polynucleotides into which the nucleotides have been built in, or oligo- and polynucleotides that have been directly modified using these novel platinum compounds may be used as probes in biomedical research, clinical diagnostics and recombinant DNA technology.

The wide variety of utilities are based upon the ability of the platinum compounds to form stable complexes with polypeptides which in turn can be detected either by means of detectable moieties which are attached to or which interact with the polypeptide. Some uses include detecting and identifying nucleic acid containing etiological agents, e.g. bacteria and viruses; screening bacteria for antibiotic resistance; screening animals for genetic disorders in relation to pharmaceutical effects; diagnosing genetic disorders, e.g. trisomy 21, sickle cell anemia: chromosomal karyotyping; and identifying tumor cells.

The invention also encompasses a diagnostic kit for detecting, determining and/or localizing biological substances of interest, comprising the platinum compound wherein the compound has a label attached thereto, optionally together with other suitable means for detection. The platinum compound in the kit may comprise one or more of a spacer, a label and a bio-organic molecule. Of course, the invention also encompasses a kit, wherein these components are present separately, i.e. in unbound form.

In a different embodiment, the invention is directed to a method for labeling bio-organic molecules wherein use is made of the compounds disclosed herein-above.

In a method for labeling a bio-organic molecule in accordance with this embodiment, a reactive moiety of a trans-platinum based compound, having the formula:

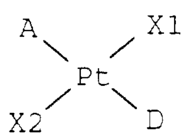

(I)

wherein A, D, X1 and X2 have the above meanings, is reacted with a label; and the other reactive moiety of said trans-platinum based compound is reacted with a bio-organic molecule or vice versa.

In a preferred method for labeling a bio-organic molecule, the label and/or bio-organic molecule are connected to the platinum through a spacer, which spacer comprises a chain having at least four atoms, which chain comprises an electron donating moiety on one end and a reactive moiety on the other end, wherein the chain is attached to the platinum through the electron donating moiety. This spacer may be any of the spacers disclosed herein-above.

Of course, the spacer(s), the label, the bio-organic molecule and the trans-platinum may be attached to each other in any order. For instance, the spacer(s) may first be attached to the platinum followed by reacting the obtained compound with label and bio-organic molecule, but it is also possible first to attach the spacer(s) to the label and/or bio-organic molecule before the reaction with the platinum compound.

In a highly preferred embodiment, the invention provides a method as described above wherein the bio-organic molecule is a nucleotide.

The invention will be elucidated by the following, non-restrictive examples.

EXAMPLE 1: Preparation of trans-diamminedichloroplatinum(II) (trans- $[Pt(NH_3)_2Cl_2]$)

1 g of potassium tetrachloroplatinate was dissolved in 20 ml Milli-Q and 0.6 ml of concentrated HCl was added. The mixture was heated until boiling and 2.5 ml of a 25% solution of $NH_4OH$ was added dropwise with swirling. The resulting solution was evaporated to about 10 ml. To the resulting pale yellow residue, 100 ml of 6N HCl was added, and the mixture was evaporated as above to a volume of 15-20 ml. The trans-diamminedichloroplatinum(II) was deposited as a fine yellow powder. After cooling in ice, the mixture was filtered, washed with three 10 ml portions of ice-cold water, ethanol and ether, and air-dried. The trans-diamminedichloroplatinum(II) was crystallized from a minimum amount of boiling 0.1 N HCl. The product was characterized by infrared spectroscopy.

EXAMPLE 2: Preparation of trans-platinum diammine-DigDadoo chloride (trans-[Pt($NH_3$)$_2$(DigDadoo-$NH_2$)Cl] ($NO_3$))

30 mg of trans-[Pt($NH_3$)$_2$$Cl_2$] (0.1 mmol) was suspended in 25 ml of N,N'-dimethylformamide and 17 mg (0.1 mmol) of $AgNO_3$ was added to the mixture which was reacted for 16 hours at room temperature shielded from the light. The precipitated AgCl was filtered off and 3.4 ml was taken from the filtrate (3.9 mg (0.014 mmol) of the trans-[Pt($NH_3$)$_2$ (Cl)($NO_3$)]) and incubated with 8 mg of DigDadoo (digoxygenin-1,8-diamino-3,6-dioxaoctane, 0.014 mmol, purchased from Boehringer Mannheim) for 48 hours at 40°C, shielded from the light. The N,N'-dimethylformamide was removed in vacuo and the remainder was stirred in $CH_2Cl_2$ whereupon a yellowish powder precipitated. The precipitated powder was filtered off, washed with $CH_2Cl_2$ and dried. The product was characterized by high resolution $^1$H-NMR.

EXAMPLE 3: Preparation of trans-platinum diammine-DigDadoo-2'-deoxyGuanosine-5'-triphosphate (trans-[Pt($NH_3$)$_2$ (DigDadoo-$NH_2$)(dGTP-N7)]$^{2-}$)

12 mg trans-[Pt($NH_3$)$_2$(DigDadoo-$NH_2$)Cl]($NO_3$) (0. 014 mmol) was dissolved in 2 ml of Milli-Q and 3 mg (0.006 mmol) of 2'-deoxyguanosine-5'-triphosphate was added. The pH of the mixture was adjusted to ~6 with 0.1 N NaOH and the reaction was performed for 24 hours at 50°C shielded from the light. The mixture was lyophilized and the residue was applied to a preparative anion exchange chromatography column (Q-Sepharose® 26/10, Pharmacia BioTech) and a gradient starting with 100% Milli-Q to 100% $NH_4HCO_3$ was applied. The appropriate fraction was collected, concentrated and lyophilized to remove $NH_4HCO_3$. The product was analyzed by high resolution $^1$H and $^{31}$P-NMR and by analytical FPLC-methods, anion exchange (MonoQ 5/5, Pharmacia BioTech) and reversed phase (PepRPC 5/5, Pharmacia BioTech). The product was converted into its tetralithium salt by passing it over a Dowex cation exchange column.

EXAMPLE 4: Preparation of trans-platinum diammine-Cy5®-chloride (trans-[Pt($NH_3$)$_2$(Cy5®-$NH_2$)Cl]($NO_3$))

7.9 mg Cy5 Monofunctional reactive dye (0.01 mmol) was dissolved in 5 ml of dry N,N'-dimethylformamide and reacted with an excess of ethylenediamine for 4 hours at room temperature in an inert atmosphere (argon). The N,N'-dimethylformamide and excess ethylenediamine were removed in vacuo. The residue was redissolved in 10 ml of N,N'-dimethylformamide and 3 mg (0.009 mmol) trans-[Pt($NH_3$)$_2$(Cl)($NO_3$)]) was added. The mixture was stirred for 16 hours at 50°C, shielded from the light. The N,N'-dimethylformamide was removed in vacuo and the residue was stirred in $CH_2Cl_2$, whereupon the product precipitated.

The precipitate was filtered off and dried *in vacuo*. The product was characterized by high resolution $^1$H-NMR.

EXAMPLE 5: Enzymatic incorporation of trans-[Pt($NH_3$)$_2$(DigDadoo-$NH_2$)(dGTP)] into pBR 322 DNA

The enzymatic incorporation was performed by incubating the following mixture overnight at 37°C:

| | |
|---|---|
| Denaturated DNA (1 µg) | 8.0 µl |
| dTTP (5.0 mM) | 0.8 µl |
| dCTP (5.0 mM) | 0.8 µl |
| dATP (5.0 mM) | 0.8 µl |
| dGTP (1.0 mM) | 2.6 µl |
| trans- [Pt ($NH_3$)$_2$(DigDadoo-$NH_2$) (dGTP)] (1 mM) | 1 .4 µl |
| High Prime Mixture* | 4.0 µl |
| Sterile demineralized water | 1.6 µl |
| Total | 20 µl |

*The High Prime Mixture contained Klenow polymerase and hexanucleotides in an optimized buffer.

The incubation was stopped by adding 2 µl of 0.2 M EDTA and the volume of the mixture was adjusted to 40 µl by adding sterile demineralized water. A filter hybridization was performed with a probe concentration of 25 ng/ml, using dentarued homologous DNA dotted on filterstrips with the following dilution range: 100pg, 30 pg, 3 pg, 1 pg, 0.3 pg, 0.1 pg, 0.03 pg, 0.01 pg and 0 pg. Detection was carried out with anti-Dig-AP (Fab fragments from an anti-digoxigening antibody from sheep conjugated with alkaline phosphatase (AP))(1:10,000) and CDP-Star (chemiluminescent substrate). As a result 0.3 pg hybridization was observed.

EXAMPLE 6 : trans-[Pt($NH_3$)$_2$(DigDadoo-$NH_2$)(dGTP)] Oligo tailing using Terminal Transferase

The tailing reaction was performed by incubating the following mixture for 15 minutes at 37°C:

| | | |
|---|---|---|
| Reaction buffer: | 1M potassium cacodylate, 0.125 M Tris-HCl, 1.25 mg/m; Bovine serum albumine pH 6.6 (25°C) | 4 µl |
| $CoCl_2$ solution (25 mM) | | 4 µl |
| oligo (20 pmol/µl) | | 5 µl |
| trans- [Pt ($NH_3$)$_2$(DigDadoo-$NH_2$)(dGTP)]/dATP* | | 1 µl |
| Terminal Transferase 50U/µl | | 1 µl |
| Sterile demineralized water | | 5 µl |
| Total | | 20 µl |

* Mixture of 9 µl of trans-[Pt($NH_3$)$_2$(DigDadoo-$NH_2$)(dGTP)] (1 mM0 and 1 µl dATP (100 mM)

The incubation was stopped by adding 2 µl of a mixture of 1 µl glycogen and 200 µl 0.2 M EDTA having a pH of 8.0. Subsequently, a purification was performed by precipitation from ethanol and a hybridization analogous to the filter hybridization described in Example 5 was performed. As a result 10 pg hybridiztion was observed.

EXAMPLE 7: Normal DNA labeling using trans-[ Pt ($NH_3$)$_2$(DigDadoo-$NH_2$)Cl]($NO_3$)

From a standard QC procedure, testing a range of different ratios, the following protocol was performed. A mixture of 2 µl DNA (2 µg), 2 µl trans-[Pt($NH_3$)$_2$(DigDadoo-$NH_2$)Cl]($NO_3$) (0.1 mg/ml) and 16 µl sterile demineralized water was incubated for 30 minutes at 85°C. The incubation was stopped by adding 5 µl 1% sodium diethyldithiocarbamic acid. In a filter hybridization, which was performed analogous to the hybridization described in Example 5, 3 pg hybridization was observed.

EXAMPLE 8: Ratio labeling of DNA by mixing painting probes that have been labeled independently

Identical painting probes were labeled in independent reactions using Fluorescein (Flu) and Rhodamine (Rho). After labeling, independent probes were mixed using a ratio of 1:9, 1:5, 1:1, 5:1 and 9:1, respectively, and tested spot blot and by filter hybridization. Detection was performed by alkaline phosphatse conjugated antibodies raised against Flu and Rho, respectively. After detection, ratios of labeled painting probes appeared to be identical when compared with the ratio of input marker molecule.

EXAMPLE 9: Ratio labeling of DNA using a defined mixed input of trans- [Pt($NH_3$)$_2$(Dadoo-$NH_2$)Cl]$NO_3$ marker complexes in one reaction

Probes were labeled using similar ratios as described in Example 8, with the exception that the probe DNA was incubated with a mixture of trans-[Pt($NH_3$)$_2$(Dadoo-$NH_2$)Cl]$NO_3$ marker (Flu and Rho) complexes in one reaction tube. After detection by conjugated antibodies, it appeared that distribution of label occurred equimolar and that different marker molecules did not interfere with each other, nor did they affect each other negatively. All results were confirmed by microscopial analysis

## Claims

**1.** A method for labeling a bio-organic molecule, wherein a reactive moiety of a trans-platinum based compound, having the formula:

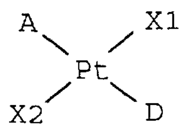

(I)

wherein A and D represent the same or different reactive moieties, and X1 and X2 represent the same or different inert moieties, is reacted with a label, and wherein the other reactive moiety of said trans-platinum based compound is reacted with a bio-organic molecule, in any order.

**2.** A method according to claim 1, wherein X1 and X2 are the same or different non-leaving ligands.

**3.** A method according to any of the preceding claims, wherein X1 and X2 are chosen from the group of $NH_3$, $NH_2R$, NHRR', NRR'R" groups, wherein R, R' and R" represent an alkyl group having from 1 to 6 carbon atoms.

**4.** A method according to any of the preceding claims, wherein X1 and X2 both represent an $N(CH_3)_3$ group.

**5.** A method according to any of the preceding claims, wherein A and/or D are chosen from the group of acetate, $NO_3^-$, $HCO_3^-$, $CO_3^{2-}$, $SO_3^-$, $Cl^-$, $I^-$, and other halogens.

**6.** A method according to any of the preceding claims, wherein the label and/or the bio-organic molecule are connected to the platinum through a spacer, which spacer comprises a chain having at least four atoms, which chain comprises an electron donating moiety on one end and a reactive moiety on the other end, wherein one or both of the reactive moieties of the trans-platinum based compound are reacted with the electron donating moiety of said spacer(s) and wherein the reactive moiety of said spacer(s) are reacted with the label and/or bio-organic molecule, in any order.

**7.** A method according to claim 6, wherein the electron donating moiety is an amine group or a thiolate anion.

**8.** A method according to claim 6 or 7, wherein the chain further comprises at least one heteroatom.

**9.** A method according to any of the claims 6-8, wherein the spacer comprises no more than 20 carbon atoms in the chain and wherein the chain is essentially non-branched.

**10.** A method according to claim 9, wherein A and/or D is 1,8-diamino-3,6-dioxaoctane.

**11.** A method according to any of the preceding claims, wherein the bio-organic molecule is chosen from the group of proteins, peptides, DNA molecules, RNA molecules, and (oligo)-nucleotides.

**12.** A method according to claim 11, wherein the bio-or ganic molecule is a nucleotide.

**13.** A method according to any of the preceding claims, wherein the label is chosen from the group biotin, avidin, streptavidin, digoxygenin, and fluorochromes including Cy®-colourants.

**14.** A trans-platinum based compound for use in a method for labeling a bio-organic molecule according to any of the preceding claims, having the formula:

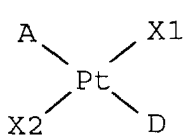

(I)

wherein A represents a reactive moiety for attachment to a label, D represents a reactive moiety for attachment to a bio-organic molecule, and X1 and X2 represent the same or different inert moieties, and wherein the compound has a label attached thereto.

**15.** A compound according to claim 14 having a bio-organic molecule attached thereto.

**16.** A compound according to claim 15, wherein the bio-organic molecule is a nucleotide.

**17.** A diagnostic kit for detecting, determining and/or localizing biological substances of interest, comprising a compound according to any of the claims 14-16, optionally together with other suitable means for detection.

## Revendications

**1.** Procédé pour marquer une molécule bioorganique, où une entité réactive d'un composé à base de trans-platine ayant la formule :

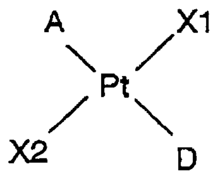

(I)

où A et D représentent des entités réactives identiques ou différentes, et X1 et X2 représentent des entités inertes identiques ou différentes, est mise à réagir avec un marqueur, et où l'autre entité réactive dudit composé à base de trans-platine est mise à réagir avec une molécule bioorganique, dans un ordre quelconque.

**2.** Procédé selon la revendication 1, où X1 et X2 sont des ligands non partants identiques ou différents.

**3.** Procédé selon l'une quelconque des revendications précédentes, où X1 et X2 sont choisis dans le groupe des groupes $NH_3$, $NH_2R$, NHRR', NRR'R", où R, R' et R" représentent un groupe alkyle ayant de 1 à 6 atomes de carbone.

**4.** Procédé selon l'une quelconque des revendications précédentes, où X1 et X2 représentent l'un et l'autre un groupe $N(CH_3)_3$.

**5.** Procédé selon l'une quelconque des revendications précédentes, où A et/ou D sont choisis dans le groupe d'acétate, $NO_3^-$, $HCO_3^-$, $CO_3^{2-}$, $SO_3$-, $Cl^-$, $I^-$ et d'autres halogènes.

**6.** Procédé selon l'une quelconque des revendications précédentes, où le marqueur et/ou la molécule bioorganique sont liés au platine par un espaceur, lequel espaceur comprend une chaîne ayant au moins 4 atomes, laquelle chaîne comprend une entité donneuse d'électrons à une extrémité et une entité réactive à l'autre extrémité, où l'une des entités réactives ou les deux entités réactives du composé à base de trans-platine sont mises à réagir avec l'entité donneuse d'électrons dudit ou desdits espaceurs et où l'entité réactive dudit ou desdits espaceurs est mise à réagir avec le marqueur et/ou la molécule bio-organique, dans un ordre quelconque.

**7.** Procédé selon la revendication 6, où l'entité de donneuse d'électrons est un groupe amine ou un anion thiolate.

**8.** Procédé selon la revendication 6 ou 7, où la chaîne comprend en outre au moins un hétéroatome.

**9.** Procédé selon l'une quelconque des revendications 6-8, où l'espaceur comprend au plus 20 atomes de carbone dans la chaîne et où la chaîne est sensiblement non ramifiée.

**10.** Procédé selon la revendication 9, où A et/ou D sont 1,8-diamino-3,6-dioxaoctane.

**11.** Procédé selon l'une quelconque des revendications précédentes, où la molécule bloorganique est choisie dans le groupe des protéines, des peptides, des molécules d'ADN, des molécules d'ARN et des (oligo)nucléotides.

**12.** Procédé selon la revendication 11, où la molécule bioorganique est un nucléotide.

13. Procédé selon l'une quelconque des revendications précédentes, où le marqueur est choisi dans le groupe de la biotine, de l'avidine, de la streptavidine, de la digoxygénine et des fluorochromes incluant les colorants Cy®.

14. Composé à base de trans-platine destiné à être utilisé dans un procédé pour marquer une molécule bioorganique selon l'une quelconque des revendications précédentes ayant la formule :

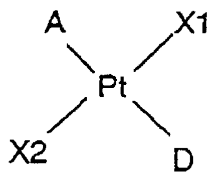

(I)

où A représente une entité réactive pour la fixation à un marqueur, D représente une entité réactive pour la fixation à une molécule bioorganique et X1 et X2 représentent des entités inertes identiques ou différentes, et où le composé a un marqueur fixé à lui.

15. Composé selon la revendication 14, ayant une molécule bioorganique fixée à lui.

16. Composé selon la revendication 15, où la molécule bioorganique est un nucléotide.

17. Trousse de diagnostique pour détecter, déterminer et/ou localiser des substances biologiques d'intérêt, comprenant un composé selon l'une quelconque des revendications 14 à 16, éventuellement avec d'autres moyens appropriés pour la détection.

**Patentansprüche**

1. Verfahren zur Markierung eines bioorganischen Moleküls, worin eine reaktive Hälfte oder Struktureinheit einer auf Trans-Platin basierenden Verbindung mit der Formel:

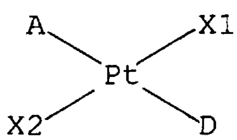

(I) ,

mit einer Markiersubstanz zur Reaktion gebracht wird, wobei A und D die gleiche oder unterschiedliche reaktive Struktureinheiten und X1 und X2 die gleiche oder unterschiedliche inerte Struktureinheiten darstellen, und worin die andere reaktive Hälfte oder Struktureinheit der auf Trans-Platin basierenden Verbindung mit einem bioorganischen Molekül zur Reaktion gebracht wird, und zwar in beliebiger Reihenfolge.

2. Verfahren nach Anspruch 1,
worin X1 und X2 die gleiche oder unterschiedliche Nicht-Überrest-Liganden darstellen.

3. Verfahren nach einem der vorhergehenden Ansprüche,
worin X1 und X2 von der Gruppe aus $NH_3$, $NH_2R$, NHRR', NHRR'R" ausgewählt sind, wobei R, R' und R" eine Alkylgruppe mit von 1 bis 6 Kohlenstoffatomen darstellen.

4. Verfahren nach einem der vorhergehenden Ansprüche, worin X1 und X2 beide eine $N(CH_3)_3$-Gruppe darstellen.

5. Verfahren nach einem der vorhergehenden Ansprüche, worin A und/oder D von der Gruppe aus Acetat, $NO_3$-, $HCO_3$-, $CO_3^{2-}$, $SO_3$-, $CL^-$, $I^-$ und anderen Halogenen ausgewählt ist.

6. Verfahren nach einem der vorhergehenden Ansprüche, worin die Markierungssubstanz und/oder das bioorganische Molekül mit dem Platin über einen Abstandshalter verbunden sind, der eine Kette mit mindestens vier Atomen

umfasst und die eine Elektronen abgebende Struktureinheit an einem Ende und eine reaktive Struktureinheit am anderen Ende aufweist, worin eine oder beide der reaktiven Struktureinheiten der auf Trans-Platin basierenden Verbindung mit der Elektronen abgebenden Struktureinheit des oder der Abstandshalter zur Reaktion gebracht werden, und worin die reaktive Struktureinheit des oder der Abstandshalter mit der Markiersubstanz und/oder dem bioorganischen Molekül in beliebiger Reihenfolge zur Reaktion gebracht werden.

**7.** Verfahren nach Anspruch 6, worin die Elektronen abgebende Struktureinheit eine Amingruppe oder ein Thiolatanion darstellen.

**8.** Verfahren nach Anspruch 6 oder 7, worin die Kette ferner mindestens ein Heteroatom umfasst.

**9.** Verfahren nach einem der Ansprüche 6 bis 8, worin der Abstandshalter nicht mehr als 20 Kohlenstoffatome in der Kette umfasst und worin die Kette im wesentlichen nicht verzweigt ist.

**10.** Verfahren nach Anspruch 9, worin A und/oder D 1,8-Diamino-3,6-dioxaoctan ist.

**11.** Verfahren nach einem der vorhergehenden Ansprüche, worin das bioorganische Molekül von der Gruppe der Proteine, Peptide, DNA-Moleküle, RNA-Moleküle und (Oligo)-Nucleotide ausgewählt ist.

**12.** Verfahren nach Anspruch 11, worin das bioorganische Molekül ein Nucleotid ist.

**13.** Verfahren nach einem der vorhergehenden Ansprüche, worin die Markiersubstanz von der Gruppe Biotin, Avidin, Streptavidin, Digoxygenin und Fluorochromen einschließlich Cy®-Färbemittel ausgewählt ist.

**14.** Auf Trans-Platin basierende Verbindung zur Anwendung in einem Verfahren zur Markierung eines bioorganischen Moleküls gemäß einem der vorhergehenden Ansprüche mit der Formel:

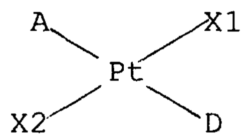

(I) ,

worin A eine reaktive Hälfte oder Struktureinheit zur Anheftung an die Markiersubstanz darstellt, D eine reaktive Hälfte oder Struktureinheit zur Anheftung an ein bioorganisches Molekül und X1 und X2 die gleiche oder unterschiedliche inerte Struktureinheiten darstellen und worin die Verbindung eine angeheftete Markierung aufweist.

**15.** Verbindung nach Anspruch 14 mit einem daran angehefteten bioorganischen Molekül.

**16.** Verbindung gemäß Anspruch 15, worin das bioorganische Molekül ein Nucleotid darstellt.

**17.** Ein diagnostischer Kit oder Testsatz zur Feststellung, Bestimmung und/oder Lokalisierung von biologischen Substanzen von Interesse, umfassend eine Verbindung gemäß einem der Ansprüche 14 bis 16, gegebenenfalls zusammen mit anderen geeigneten Nachweismitteln.